# EUROPÄISCHE PATENTSCHRIFT

(11) **EP 2 245 189 B1**
(45) Veröffentlichungstag und Bekanntmachung des Hinweises auf die Patenterteilung: **18.09.2013**
(21) Anmeldenummer: 08854644.5
(22) Anmeldetag: 28.11.2008
(51) Int. Cl.: C12Q 1/68

(54) **STEGANOGRAPHISCHE EINBETTUNG VON INFORMATIONEN IN KODIERENDEN GENEN**
STEGANOGRAPHIC EMBEDDING OF INFORMATION IN CODING GENES
INTÉGRATION STÉGANOGRAPHIQUE D'INFORMATIONS DANS DES GÈNES CODANTS

(30) Priorität: 30.11.2007 DE 102007057802
(43) Veröffentlichungstag der Anmeldung: 03.11.2010
(73) Patentinhaber: GENEART AG, 93053 Regensburg (DE)
(72) Erfinder: LISS, Michael, 93059 Regensburg (DE)
(74) Vertreter: Wöhler, Christian
(86) Internationale Anmeldenummer: PCT/EP2008/010128
(87) Internationale Veröffentlichungsnummer: WO 2009/068305

(56) Entgegenhaltungen:
- EP-A- 1 568 783
- WO-A-00/68431
- ARITA MASANORI ET AL: "Secret signatures inside genomic DNA" BIOTECHNOLOGY PROGRESS, Bd. 20, Nr. 5, September 2004 (2004-09), Seiten 1605-1607, XP002524663 ISSN: 8756-7938
- LEIER A ET AL: "Cryptography with DNA binary strands" BIOSYSTEMS, NORTH-HOLLAND, AMSTERDAM, NL, Bd. 57, Nr. 1, 1. Juni 2000 (2000-06-01), Seiten 13-22, XP002305546 ISSN: 0303-2647

## Beschreibung

Die vorliegende Erfindung betrifft die Speicherung von Informationen in Nukleinsäuresequenzen. Weiterhin beschrieben sind Nukleinsäuresequenzen, in denen gewünschte Informationen enthalten sind, sowie den Entwurf, die Herstellung oder Verwendung solcher Sequenzen.

Wichtige Informationen, vor allem geheime Informationen, müssen vor unbefugtem Zugriff geschützt werden. Zu diesem Zweck wurden in der Vergangenheit immer ausgefeiltere kryptographische oder steganographische Techniken entwickelt. Es existieren zahlreiche Algorithmen zur Verschlüsselung von Daten und zur Tarnung geheimer Informationen. Die Sicherheit einer geheimen steganographischen Information beruht u.a. darauf, dass ihre Existenz einem Unbefugten nicht auffällt. Die Information wird in einem unauffälligen Medium verpackt, wobei das Medium im Prinzip frei gewählt werden kann. Beispielsweise ist es im Stand der Technik bekannt, Informationen in digitalen Bildern oder Audiodateien zu verbergen. Ein Pixel eines digitalen RGB-Bildes besteht aus 3 x 8 bit. Je 8 bit kodieren für die Helligkeit des Rot-, Grün- und Blaukanals. Jeder Kanal kann 256 Helligkeitsstufen aufnehmen. Wird das letzte bit (least significant bit, LSB) jedes Pixels und Kanals mit einer fremden Information überschrieben, so ändert sich die Helligkeit jedes Kanals nur um 1/256, also um 0,4 %. Das Bild sieht für einen Betrachter immer noch unverändert aus.

Musik auf CD ist mit 44.100 Samples/Sekunde, 2 Kanälen, 16 bit/Sample digitalisiert. Bei einer Überschreibung des LSB eines Samples ändert sich die Wellenamplitude an dieser Stelle um 1/65536, also 0,002 %. Diese Änderung ist für Menschen nicht hörbar. Eine herkömmliche CD bietet somit Platz für 74 min x 60 sec x 44.100 Samples x 2 Kanäle = 392 Mbit oder ~50 Mbyte.

In den letzten Jahren wurden außerdem Steganographie-Ansätze auf Basis der DNA entwickelt. Clelland et al. (Nature 399:533-534 und US 6,312,911) entwickelten, Inspiriert von den im zweiten Weltkrieg verwendeten Mikropunkten, ein Verfahren zum Verbergen von Nachrichten in so genannten DNA-Microdots. Sie erzeugten künstliche DNA-Stränge, die aus einer Abfolge von Tripletts zusammengesetzt waren, denen jeweils ein Buchstabe oder eine Zahl zugeordnet wurde. Dem Empfänger der geheimen Information müssen dann für die Decodierung der Nachricht die Primer für Amplifizierung und Sequenzierung sowie der Dechiffriercode bekannt sein.

US-Patent 6,537,747 offenbart Verfahren zur Verschlüsselung von Informationen aus Worten, Zahlen oder graphischen Bildern. Die Information wird direkt in Nukleinsäurestränge eingebaut, die an den Empfänger übermittelt werden, der die Information unter Verwendung eines Schlüssels dekodieren kann.

Die von Clelland und in US 6,537,747 beschriebenen Verfahren beruhen jeweils auf der direkten Speicherung von Informationen in DNA. Der Nachteil einer solchen direkten Speicherung über einen einfachen Triplett-Code ist jedoch, dass auf diese Weise auffällige Sequenzmotive entstehen können, die von Dritten bemerkt werden könnten. Sobald erkannt wird, dass in einem Medium eine geheime Information enthalten ist, besteht die Gefahr, dass diese Information auch entschlüsselt wird. Weiterhin können solche DNA-Bereiche nur sehr bedingt eine biologisch relevante Funktion erfüllen. Bei der Erzeugung gentechnisch veränderter Organismen müssen die Nukleinsäuren, die die verschlüsselte Botschaft enthalten, also zusätzlich zu den Genen eingebracht werden, die die gewünschten Eigenschaften des Organismus hervorrufen.

Arita und Ohashi (Biotechnol. Prog. (2004) 20: 1605-1607) beschreiben ein Verfahren, um genomische bzw. wildtypische DNA-Sequenzen mit Wasserzeichen zu versehen. Kodons, die im genetischen Code degeneriert sind, werden gegen ein alternatives Kodon für dieselbe Aminosäure ausgetauscht, um im Binärcode den Wert 1 zu vermitteln, während das Beibehalten des ursprünglichen, in der Wildtyp-Sequenz enthaltenen Kodons den binären Wert 0 vermittelt.

Es war daher die Aufgabe der vorliegenden Erfindung, ein verbessertes steganographisches Verfahren zur Einbettung von Informationen in Nukleinsäuren bereitzustellen, weiches noch sicherer gegenüber einer unerwünschten Entschlüsselung ist. Die Information sollte dabei so verborgen werden, dass für einen Dritten nicht erkennbar ist, dass überhaupt eine geheime Information enthalten ist.

Die Erfinder der vorliegenden Erfindung haben herausgefunden, dass die Degeneriertheit des genetischen Codes genutzt werden kann, um Informationen in kodierenden Nukleinsäuren einzubetten. Unter der Degeneriertheit des genetischen Codes versteht man, dass eine bestimmte Aminosäure von unterschiedlichen Kodons kodiert sein kann. Als Kodon bezeichnet man die Abfolge einer Sequenz von drei Nukleobasen, die im genetischen Code für eine Aminosäure kodiert. Erfindungsgemäß wurde ein Verfahren entwickelt, mit dem Nukleinsäuresequenzen bereitgestellt werden, die so modifiziert sind, dass eine gewünschte Information enthalten ist.

Gegenstand der Erfindung ist in einem ersten Aspekt ein Verfahren zum Entwerfen von Nukleinsäuresequenzen, in denen Informationen enthalten sind, welches die Schritte umfasst:
(a) Zuordnen eines ersten bestimmten Werts für mindestens ein erstes Nukleinsäure-Kodon aus einer Gruppe von degenerierten NukleinsäureKodons, welche dieselbe Aminosäure kodieren,
   Zuordnen eines zweiten bestimmten Werts für mindestens ein zweites Nukleinsäure-Kodon aus der Gruppe,
   gegebenenfalls Zuordnen eines oder mehrerer weiterer bestimmter Werte für jeweils mindestens ein weiteres Nukleinsäure-Kodon aus der Gruppe,
   worin die ersten und zweiten und ggf. weitere Werte innerhalb der Gruppe von Kodons welche dieselbe Aminosäure kodieren mindestens jeweils einmal vergeben werden;
(b) Bereitstellen einer zu speichernden Information als Abfolge von n Werten, die jeweils ausgewählt sind aus ersten und zweiten und ggf. weiteren Werten, worin n eine ganze Zahl ≥ 1 ist;
(c) Bereitstellen einer Ausgangs-Nukleinsäuresequenz, wobei die Sequenz n degenerierte Kodons umfasst, denen gemäß (a) erste und zweite und gegebenenfalls weitere Werte zugeordnet sind, worin n eine ganze Zahl ≥ 1 ist; und
(d) Entwerfen einer modifizierten Sequenz der Nukleinsäure aus (c), worin an den Positionen der n degenerierten Kodons der Ausgangs-Nukleinsäuresequenz jeweils ein Nukleinsäure-Kodon aus der Gruppe degenerierter Kodons, die für dieselbe Aminosäure kodieren, ausgewählt wird, dem durch die Zuordnung aus (a) ein Wert entspricht, so dass die Abfolge der den n Kodons zugeordneten Werten die zu speichernde Information ergibt.

Im genetischen Code stehen insgesamt 64 unterschiedliche Kodons zur Verfügung, die insgesamt für 20 verschiedene Aminosäuren sowie für Stopp kodieren. (Auch Stoppkodons sind prinzipiell für die Unterbringung von Informationen geeignet.) Für manche Aminosäuren sowie Stopp werden daher mehrere Kodons verwendet. Beispielsweise sind die Aminosäuren Tyr, Phe, Cys, Asn, Asp, Gln, Glu, His und Lys jeweils zweifach kodiert. Für die Aminosäure Ile und für Stopp existieren jeweils drei degenerierte Kodons. Die Aminosäuren Gly, Ala, Val, Thr und Pro sind jeweils vierfach kodiert und die Aminosäuren Leu, Ser und Arg sind jeweils sechsfach kodiert. Die unterschiedlichen Kodons, die für dieselbe Aminosäure kodieren, unterscheiden sich in der Regel nur in einer der drei Basen. Meist unterscheiden sich die betroffenen Kodons in der dritten Base eines Kodons.

In Schritt (a) des erfindungsgemäßen Verfahrens wird diese Degeneriertheit des genetischen Codes ausgenutzt, um degenerierten Nukleinsäurekodons innerhalb einer Gruppe von Kodons, welche dieselbe Aminosäure kodieren, bestimmte Werte zuzuordnen. In Schritt (a) wird innerhalb einer Gruppe von degenerierten Nukleinsäurekodons, welche dieselbe Aminosäure kodieren, mindestens einem ersten Nukleinsäurekodon ein erster bestimmter Wert zugeordnet und mindestens einem zweiten Nukleinsäurekodon aus dieser Gruppe wird ein zweiter bestimmter Wert zugeordnet. Dabei werden die ersten und zweiten Werte innerhalb der Gruppe von Kodons, welche dieselbe Aminosäure kodieren, mindestens jeweils einmal vergeben.

Diese Zuordnung kann für eine oder mehrere der mehrfach kodierten Aminosäuren erfolgen. Grundsätzlich kann eine solche Zuordnung für alle mehrfach kodierten Aminosäuren erfolgen. Bevorzugt erfolgt eine Zuordnung nur für die mindestens dreifach, vorzugsweise mindestens vierfach, weiter bevorzugt sechsfach kodierten Aminosäuren. Es ist erfindungsgemäß besonders bevorzugt, nur den Kodons der vierfach kodierten Aminosäuren und/oder den Kodons der sechsfach kodierten Aminosäuren bestimmte Werte zuzuordnen.

Wenn auch die zweifach kodierten Aminosäuren in die Zuordnung in Schritt (a) einbezogen werden, so kann nur eine Zuordnung von einem ersten und einem zweiten Wert erfolgen. Werden nur die mindestens vierfach kodierten Aminosäuren einbezogen, so können innerhalb einer Gruppe von degenerierten Nukleinsäurekodons, welche dieselbe Aminosäure kodieren, insgesamt bis zu vier unterschiedliche Werte vergeben werden. Wenn nur sechsfach kodierte Aminosäuren einbezogen werden, so können innerhalb einer Gruppe degenerierter Nukleinsäurekodons bis zu sechs verschiedene Werte vergeben werden.

Durch die Zuordnung von mehr als zwei, d.h. insbesondere von vier oder sechs unterschiedlichen Werten innerhalb einer Gruppe, kann über eine kürzere Abfolge von Kodons eine größere Informationsmenge gespeichert werden. In einer erfindungsgemäßen Ausführungsform ist es daher vorgesehen, in Schritt (a) nur den Kodons solcher Aminosäuren, die mindestens vierfach, vorzugsweise sechsfach kodiert sind, Werte zuzuordnen. Innerhalb der Gruppe von degenerierten Nukleinsäurekodons, welche dieselbe mehrfach kodierte Aminosäure kodieren, werden dann vorzugsweise erste und zweite und ein oder mehrere weitere Werte für jeweils mindestens ein Nukleinsäurekodon aus der Gruppe zugeordnet. Die ersten und zweiten und gegebenenfalls weitere Werte werden innerhalb der Gruppe von Kodons mindestens jeweils einmal vergeben.

Wenn nur die mindestens vierfach bzw. sechsfach kodierten Aminosäuren in die Zuordnung von Schritt (a) einbezogen werden, so ist es alternativ auch möglich, innerhalb einer Gruppe von degenerierten Nukleinsäurekodons, welche die dieselbe Aminosäure kodieren, einen ersten bestimmten Wert mehr als einem ersten Nukleinsäurekodon, d.h. zwei, drei, vier oder fünf Nukleinsäurekodons zuzuordnen und/oder einen zweiten bestimmten Wert mehr als einem zweiten Nukleinsäurekodon aus der Gruppe zuzuordnen, d.h. zwei, drei, vier oder fünf Nukleinsäurekodons. Vorzugsweise werden die ersten und zweiten Werte innerhalb der Gruppe degenerierter Kodons jeweils mehrfach, vorzugsweise gleich oft vergeben. Das heißt, innerhalb einer Gruppe von degenerierten Nukleinsäurekodons, welche dieselbe vierfach kodierte Aminosäure kodieren, wird vorzugsweise zwei Nukleinsäurekodons ein erster Wert zugeordnet und zwei anderen Kondons wird ein zweiter Wert zugeordnet. Entsprechend wird bei Einbeziehung von sechsfach kodierten Aminosäuren vorzugsweise drei Nukleinsäurekodons aus einer Gruppe ein erster Wert zugeordnet und drei anderen Nukleinsäurekodons, welche dieselbe Aminosäure kodieren, wird ein zweiter Wert zugeordnet. Auf diese Weise stehen für jeden ersten und für jeden zweiten Wert mindestens zwei mögliche Kodons, welche dieselbe Aminosäure kodieren, zur Verfügung. Die Alternative von mehreren möglichen Kodons für einen bestimmten Wert erlaubt die Vermeidung unerwünschter Sequenzmotive.

In einer bevorzugten Ausführungsform der Erfindung wird allen Nukleinsäurekodons aus einer Gruppe von degenerierten Nukleinsäurekodons, welche dieselbe Aminosäure kodieren, in Schritt (a) ein bestimmter Wert zugeordnet. Es ist erfindungsgemäß jedoch auch möglich, nur einzelnen der degenerierten Nukleinsäurekodons einen Wert zuzuordnen und andere Nukleinsäurekodons, welche dieselbe Aminosäure kodieren, nicht zu berücksichtigen.

In Schritt (b) des erfindungsgemäßen Verfahrens wird eine zu speichernde Information als Abfolge von n Werten bereitgestellt, die jeweils aus ersten und zweiten und gegebenenfalls weiteren Werten ausgewählt sind. n ist dabei eine ganze Zahl ≥ 1. Bei der zu speichernden Information kann es sich beispielsweise um Grafik, Text- oder Bilddaten handeln. Die zu speichernde Information kann in Schritt (b) auf beliebige Weise als Abfolge von n Werten bereitgestellt werden. Dabei ist zu beachten, dass die n Werte ausgewählt sind aus denselben ersten und zweiten und gegebenenfalls weiteren Werten, die in Schritt (a) bestimmten Nukleinsäurekodons zugeordnet werden. Wenn also in Schritt (a) beispielsweise nur erste und zweite Werte zugeordnet werden, so muss die zu speichernde Information in Schritt (b) als Abfolge von Werten bereitgestellt werden, die ausgewählt sind aus diesen ersten und zweiten Werten. Die zu speichernde Information wird so in binärer Form bereitgestellt. Hierfür können beispielsweise Textdaten mittels des im Fachbereich bekannten ASCII-Codes in binärer Form dargestellt werden. Wenn in Schritt (a) zusätzlich zu den ersten und zweiten Werten auch ein oder mehrere weitere Werte zugeordnet werden, so kann die zu speichernde Information in Schritt (b) als Abfolge von n Werten bereitgestellt werden, die ausgewählt sind aus ersten und zweiten und diesen weiteren Werten.

In einer bevorzugten Ausführungsform wird die zu speichernde Information nicht direkt in eine Abfolge von n Werten umgewandelt, sondern zuvor auf beliebige bekannte Weise verschlüsselt. Erst die verschlüsselte Information wird dann wie oben beschrieben in eine Abfolge von n Werten umgewandelt. Hierfür verwendbare Verschlüsselungsalgorithmen sind im Stand der Technik bekannt, wie beispielsweise Cäsar-Chiffre, Data Encryption Standard, One-Time-Pad, Vigenere, Rijndael, Twofish, 3DES. (Literatur zu Verschlüsselungsalgorithmen: Bruce Schneier: Angewandte Kryptographie. John Wiley & Sons, 1996, ISBN 0-471-1109-9).

In Schritt (c) des erfindungsgemäßen Verfahrens wird eine Ausgangs-nukleinsäuresequenz bereitgestellt. Die Ausgangsnukleinsäuresequenz kann beliebig gewählt werden. Beispielsweise kann die Nukleinsäuresequenz eines natürlich vorkommenden Polynukleotids verwendet werden. Unter dem Begriff "Polynukleotid" wird erfindungsgemäß ein aus mehreren Nukleotiden aufgebautes Oligomer oder Polymer verstanden. Die Länge der Sequenz ist durch die Verwendung des Begriffs Polynukleotid in keiner Weise begrenzt, sondern umfasst erfindungsgemäß eine beliebige Zahl von Nukleotideinheiten. Die Ausgangsnukleinsäuresequenz ist erfindungsgemäß ein kodierender DNA-Strang. Die Ausgangsnukleinsäuresequenz ist bevorzugt eine natürlich vorkommende kodierende DNA-Sequenz, die für ein bestimmtes Protein kodiert.

Die Ausgangsnukleinsäuresequenz umfasst n degenerierte Kodons, denen gemäß (a) erste und zweite und gegebenenfalls weitere Werte zugeordnet sind. n ist eine ganze Zahl ≥ 1 und entspricht der Anzahl der n Werte der zu speichernden Information aus Schritt (b). Die n degenerierten Kodons können in der Ausgangsnukleinsäuresequenz wahlweise unmittelbar aufeinander folgend angeordnet sein oder ihre Abfolge kann von anderen nicht-degenerierten Kodons oder degenerierten Kodons, denen gemäß (a) kein Wert zugeordnet ist, unterbrochen sein. Außerdem ist es möglich, dass die Abfolge der n degenerierten Kodons an einer oder mehreren Stellen von nicht kodierenden Bereichen unterbrochen ist. In einer bevorzugten Ausführungsform sind die n degenerierten Kodons in einer nicht unterbrochenen kodierenden Sequenz enthalten. Besonders bevorzugt kodiert die Ausgangsnukleinsäure für ein bestimmtes Polypeptid.

In Schritt (d) des erfindungsgemäßen Verfahrens wird eine modifizierte Sequenz der Nukleinsäuresequenz aus (c) entworfen. In der modifizierten Sequenz werden an den Positionen der n degenerierten Kodons der Ausgangsnukleinsäuresequenz jeweils Nukleinsäurekodons aus der Gruppe degenerierter Kodons, die für dieselbe Aminosäure kodieren, ausgewählt, denen durch die Zuordnung aus (a) ein Wert zugeordnet wurde. Die Auswahl der degenerierten Kodons erfolgt derart, dass die Abfolge der den n Kodons zugeordneten Werten die zu speichernde information ergibt. Wenn die Ausgangsnukleinsäuresequenz für ein Polypeptid kodiert, so kodiert die in Schritt (d) entworfene modifizierte Sequenz vorzugsweise für dasselbe Polypeptid. Unter dem Begriff "Polypeptid" wird erfindungsgemäß eine Aminosäurekette mit beliebiger Länge verstanden.

In einer erfindungsgemäßen Ausführungsform kann der Anfang und/oder das Ende einer Information in der modifizierten Sequenz aus Schritt (d) markiert werden, indem ein vereinbartes Stoppzeichen eingebaut wird. Beispielsweise kann sich an die Abfolge der n Kodons, welche die zu speichernde Information ergeben, eine Abfolge mehrerer Kodons, denen derselbe Wert zugeordnet ist, anschließen.

In einer besonders bevorzugten Ausführungsform erfolgt in Schritt (a) die Zuordnung eines ersten oder zweiten oder gegebenenfalls weiteren Wertes zu einem Nukleinsäurekodon innerhalb der Gruppe von degenerierten Kodons, welche dieselbe Aminosäure kodieren, abhängig von der Häufigkeit des Gebrauchs des Kodons in einem bestimmten Organismus. Anhand einer speziesspezifischen Kodon Usage Tabelle (CUT) können verschiedenen degenerierten Kodons unterschiedliche Werte zugeordnet werden. Beispielsweise kann innerhalb einer Gruppe von degenerierten Nukleinsäurekodons, welche dieselbe Aminosäure kodieren, dem erstbesten Kodon, d.h. dem von einer Spezies am häufigsten gebrauchten Kodon, ein erster Wert zugeordnet werden und einem zweitbesten Kodon kann ein zweiter Wert zugeordnet werden. Wenn nur die mindestens vierfach oder sechsfach kodierten Aminosäuren in die Zuordnung von Schritt (a) einbezogen werden, so können auf diese Weise ein oder mehrere weitere Werte innerhalb der Gruppe degenerierter Kodons, welche dieselbe Aminosäure kodieren, vergeben werden. In einer bevorzugten Ausführungsform werden innerhalb der Gruppe nur erste und zweite Werte vergeben. Beispielsweise wird in einer Ausführungsform dem ersten und dem drittbesten Kodon ein erster Wert zugeordnet und dem zweiten und dem viertbesten Kodon wird ein zweiter Wert zugeordnet. Es sind erfindungsgemäß beliebige Arten von Zuordnungen möglich, solange innerhalb einer Gruppe degenerierter Kodons, welche dieselbe Aminosäure kodieren, mindestens ein erster und mindestens ein zweiter Wert zugeordnet wird.

Durch die Alternative von mehreren möglichen Kodons pro Wert innerhalb einer Gruppe degenerierter Kodons ist es möglich, bei dem Entwerfen einer modifizierten Sequenz in Schritt (d) unerwünschte Sequenzmotive zu vermeiden.

Wenn in einer speziesspezifischen Kodon Usage Tabelle zwei oder mehr Kodons die gleiche Häufigkeit aufweisen, so wird für die Zuordnung von Werten eine weitere Bedingung vereinbart.

Alternativ zu der Zuordnung von Werten auf Grundlage der Häufigkeit des Gebrauchs eines Kodons innerhalb einer Gruppe degenerierter Kodons oder als weitere Bedingung, wie oben erwähnt, kann eine Zuordnung auch auf Basis einer alphabetischen Sortierung erfolgen. Darüber hinaus sind zahlreiche weitere Möglichkeiten der Zuordnung denkbar und die vorliegende Erfindung soll nicht auf die Zuordnung auf Basis der Häufigkeit des Kodongebrauchs beschränkt sein.

Die in Schritt (d) entworfene modifizierte Nukleinsäuresequenz kann in einer besonders bevorzugten Ausführungsform des erfindungsgemäßen Verfahrens in einem anschließenden Schritt (e) hergestellt werden. Die Herstellung kann nach beliebigen, im Fachbereich bekannten Verfahren erfolgen. Beispielsweise kann eine Nukleinsäure mit der in Schritt (d) entworfenen modifizierten Sequenz durch Mutation aus der Ausgangssequenz von Schritt (c) hergestellt werden. Insbesondere eignet sich hierfür erfindungsgemäß eine Substitution einzelner Nukleobasen. Ebenfalls möglich ist die Mutation durch Insertionen und Deletionen. Außerdem ist es möglich, in Schritt (e) eine Nukleinsäure mit der modifizierten Sequenz synthetisch herzustellen. Verfahren zur Herstellung synthetischer Nukleinsäuren sind dem Fachmann bekannt.

Das erfindungsgemäße Verfahren führt zu einer modifizierten Nukleinsäuresequenz, in der eine gewünschte Information verschlüsselt enthalten ist. Der Schlüssel dafür liegt in der Zuordnung von Schritt (a). Dieser Schlüssel muss einem Adressaten der Information bekannt sein. Beispielsweise kann der Schlüssel dem Adressaten separat zu einem gesonderten Zeitpunkt übermittelt werden.

In einer besonders bevorzugten Ausführungsform kann der Schlüssel für die Zuordnung gemäß (a) selbst verschlüsselt und in einer Nukleinsäure gespeichert werden. Beispielsweise kann der Schlüssel zusätzlich in die in dem erfindungsgemäßen Verfahren erhaltene modifizierte Nukleinsäuresequenz eingebaut werden oder separat in eine andere Nukleinsäure eingebaut werden. Die Verschlüsselung des Schlüssels für die Zuordnung von (a) erfolgt im Allgemeinen nach einem anderen Schlüssel. Hierfür können grundsätzlich bekannte Methoden des Standes der Technik eingesetzt werden. Damit der in einer Nukleinsäure hinterlegte Schlüssel auffindbar ist, wird er vorzugsweise an einer vereinbarten Stelle untergebracht, beispielsweise unmittelbar hinter einem Stoppkodon, hinter der 3'-Klonierungsstelle oder ähnlichem. Möglich ist auch die Unterbringung an ganz anderer Stelle innerhalb des Genoms oder episomal. Über die Flankierung der Schlüsselsequenz mit spezifischen (nur Eingeweihten bekannten) Primerbindungsstellen ist dieser Schlüssel dann nur über eine spezifische PCR und Sequenzierung des PCR-Produkts zugänglich. Außerdem ist es vorteilhaft, auch die hinterlegte Schlüsselsequenz selbst mit einem Passwort zu verschlüsseln, damit sie nicht als solche erkennbar ist. Hierfür verwendbare Verschlüsselungsalgorithmen sind im Stand der Technik bekannt, beispielsweise Cäsar-Chiffre, Data Encryption Standard, One-Time-Pad, Vigenère, Rijndael, Twofish, 3DES. (Literatur zu Verschlüsselungs-algorithmen: Bruce Schneier: Angewandte Kryptographie. John Wiley & Sons, 1996, ISBN 0-471-11709-9).

Verfahren zur Herstellung von Nukleinsäuren sind dem Fachmann bekannt. Beispielsweise kann die Herstellung auf Basis von Phosphoramidit-Chemie, durch Chip-basierte Syntheseverfahren oder Festphasensyntheseverfahren erfolgen. Selbstverständlich können darüber hinaus aber auch beliebige andere Syntheseverfahren angewendet werden, die dem Fachmann geläufig sind.

Gegenstand der Erfindung ist ferner ein Verfahren, wonach die in Schritt(e) hergestellte modifizierte Nukleinsäure in einen Vektor eingebracht wird. Verfahren zur Insertion von Nukleinsäuren in einen beliebigen geeigneten Vektor sind dem Fachmann bekannt.

Die Erfindung betrifft weiter ein Verfahren, wonach die modifizierte Nukleinsäure oder der Vektor in eine Zelle eingebracht wird.

In einer weiteren Ausführungsform betrifft die vorliegende Erfindung ein Verfahren weiterhin umfassed den Schritt Übermittlung einer gewünschten Information, worin die Nukleinsäuresequenz, Nukleinsäure, der Vektor, und/oder die Zelle an einen gewünschten Empfänger übermittelt wird. Es ist besonders bevorzugt, vor der Übermittlung an den Empfänger die Nukleinsäuren, den Vektor, bzw. die Zelle mit anderen Nukleinsäuren, Vektoren, bzw. Zellen zu vermischen, welche die gewünschte Information nicht enthalten. Diese so genannten Attrappen können beispielsweise keinerlei Informationen enthalten oder andere Informationen enthalten, welche der Ablenkung dienen und nicht die gewünschte Information darstellen.

Weiter kann die in einer modifizierten Nukleinsäuresequenz enthaltende Information auch als "Wasserzeichen" für die Markierung eines Gens, oder einer Zelle dienen. Gegenstand der Erfindung ist daher in einer Ausführungsform ein Verfahren, wobei die in Schritt(e) hergestellte modifizierten Nukleinsäuresequenz zur Kennzeichnung eines Gens, und/oder einer Zelle verwendet wird. Die Markierung von Genen, bzw. Zellen mit einem erfindungsgemäßen Wasserzeichen erlaubt deren eindeutige Identifizierung. Herkunft und Echtheit können so eindeutig bestimmt werden. Für die Kennzeichnung eines Gens, oder einer Zelle mit einem erfindungsgemäßen "Wasserzeichen" wird eine natürliche Nukleinsäuresequenz des Gens bzw. der Zelle oder ein Teil der Sequenz wie vorstehend beschrieben modifiziert. An den Positionen von degenerierten Kodons der Ausgangssequenz werden jeweils Kodons, die für dieselbe Aminosäure (bzw. ebenfalls für Stopp) kodieren, ausgewählt, denen ein bestimmter Wert zugeordnet wurde. Die Auswahl der Kodons erfolgt derart, dass die Abfolge der ihnen zugeordneten Werte in der Nukleinsäuresequenz einem spezifischen Kennzeichen entspricht. Für einen Dritten ist diese Markierung nicht erkennbar; die Funktion des Gens, bzw. der Zelle wird nicht beeinträchtigt.

Die Erfindung wird durch die folgenden Figuren und Beispiele weiter veranschaulicht.

### FIGUREN

- **Figur 1**:: Ausschnitt aus der internationalen ASCII-Tabelle.

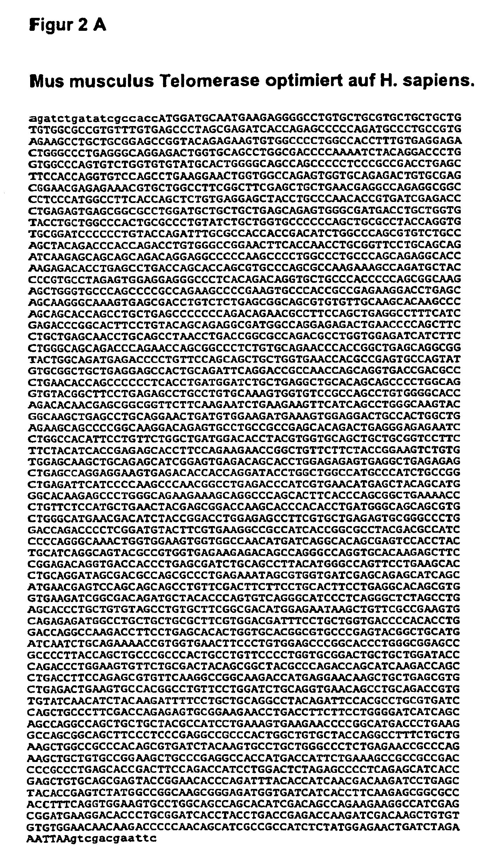

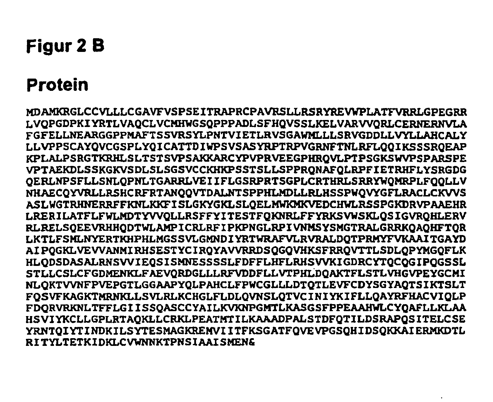

- **Figur 2**: zeigt das in Beispiel 1 verwendete Testgen (Maustelomerase), optimiert auf H. Sapiens (A) und das kodierte Protein (B)

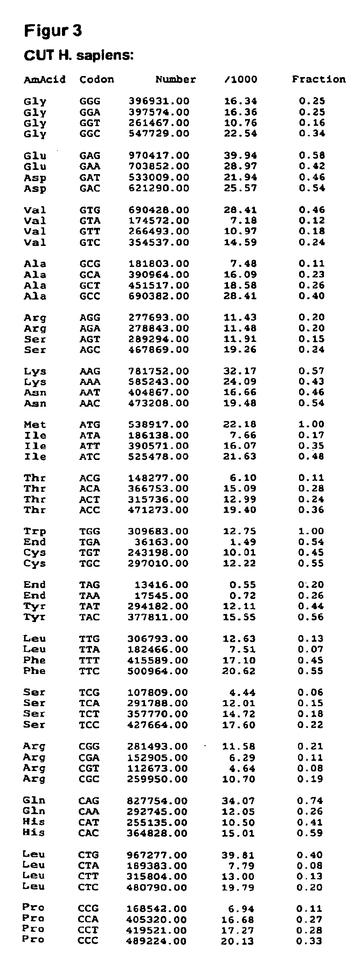

- **Figur 3**:: Kodon Usage Tabelle (CUT) für Homo Sapiens

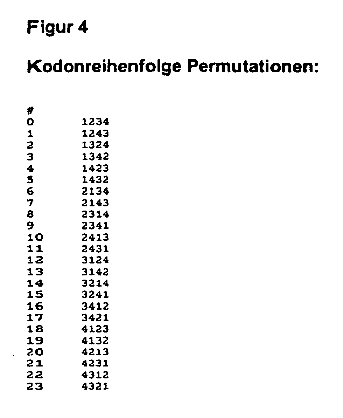

- **Figur 4**:: Kodonreihenfolge der Permutationen
- **Figur 5**: zeigt eine Analyse der in Beispiel 1 erhaltenen modifizierten Sequenz im Vergleich zur Ausgangssequenz
- **Figur 6**: zeigt ein Alignment der Sequenzen von eGFP(opt) und eGFP (msg) aus Beispiel 3. Die translatierte Aminosäuresequenz des Proteins eGFP ist oberhalb des Alignments wiedergegeben. Stille Substitutionen, die durch die Verwendung alternativer Codons bei der Einbettung der Nachricht "AEQUOREA VICTORIA." in eGFP(msg) entstanden, sind schwarz hinterlegt. Klonierungsschnittstellen sind unterstrichen, downstream der 3' HindIII Schnittstelle ist der Vektor-Anteil des 6xHis-tag mit angegeben.
- **Figur 7**: zeigt die Ergebnisse der Analyse der Expression der Gene eGFP (opt) und eGFP(msg) aus Beispiel 3 über Coomassie-Gel, Western Blot (mit einem GFP-spezifischen Antikörper) und Fluoreszenzanalyse.
- **Figur 8**: zeigt ein Alignment der Sequenzen von EMG1 (opt), EMG1 (msg) und EMG1(enc) aus Beispiel 4. Die translatierte Aminosäuresequenz des Proteins EMG1 ist oberhalb des Alignments wiedergegeben. Stille Substitutionen, die durch die Verwendung alternativer Codons bei der Einbettung der Nachricht "*GENEART AG* PAT US1234567" in EMG1(msg) und der verschlüsselten Nachricht "*: JQWF&G%DY%$4Y#'XE%87G; K*" in EMG1(enc) entstanden, sind schwarz hinterlegt. Klonierungsschnittstellen sind unterstrichen.
- **Figur 9**: zeigt das Ergebnis der Analyse der Expression von EMG1 (opt), EMG1(msg) und EMG1(enc) mittels Western Blot-Analyse unter Verwendung eines His-spezifischen Antikörpers.

### BEISPIELE

### Beispiel 1: Verschlüsselung von "GENE" im N-Terminus der Telomerase von M. musculus (optimiert auf H. Sapiens)

Als Träger für die Verschlüsselung der Nachricht "GENE" wurde der N-Terminus der Telomerase vom M. musculus gewählt. M. Musculus-Telomerase (1251AA) umfasst 360 vierfach degenerierte, informationsenthaltende Kodons (ICCs) und 372 sechsfach degenerierte ICCs. Der offene Leserahmen des Gens (ORF) wird zunächst auf übliche Weise optimiert, d.h. die Kodonwahl wird an die spezifischen Gegebenheiten des Zielorganismus angepasst.

Im Folgenden werden nur die Codons betrachtet, die 4- und 6-fach degeneriert sind, also für die Aminosäuren VPTAG (je 4 Kodons) und LSR (je 6 Kodons). Diese werden ICC (information containing codons) genannt. (Auch Aminosäuren, für die nur 2 oder 3 Kodons existieren (DEKNIQHCYF) können prinzipiell genutzt werden. Da hierbei aber die Performance des Gens stärker leidet, wird in diesem Beispiel davon abgesehen.)

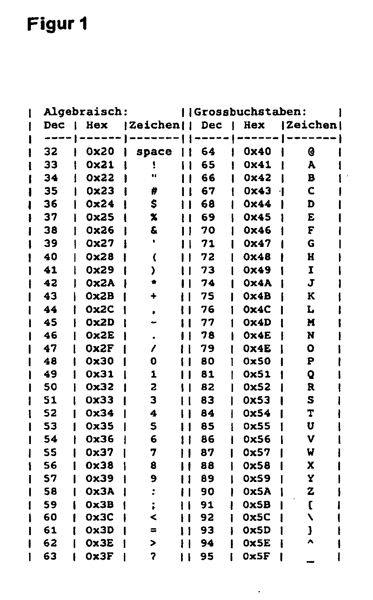

Die geheime Information (u.U. vorher verschlüsselt) wird nun in bits zerlegt. Dabei sind 6 bit (=2⁶=64 Zustände) pro Zeichen ausreichend für Buchstaben + Ziffern + Sonderzeichen. Idealerweise die ASCII-Zeichen von 32 = 0010 0000 (space) bis 95=0101 1111 (underscore). Dieser Bereich beinhaltet die Großbuchstaben, die Ziffern sowie die wichtigsten Sonderzeichen (siehe Figur 1). Die Reduktion des achtstelligen ASCII-Codes auf einen 6bit Code erfolgt nach der gängigen Bitoperation: 6 bit = 8 bit - 32 bzw. 8 bit = 6 bit + 32.

In diesem Beispiel wird für die Verschlüsselung die nachstehende CUT für Homo sapiens verwendet:

| ICC CUT *H. sapiens* (sortiert nach "Fraction" (1) & alphabetisch (2)) | | | | | | | | | | | |
|---|---|---|---|---|---|---|---|---|---|---|---|
| AA | Codon | Fraction | AA | Codon | Fraction | AA | Codon | Fraction | AA | Codon | Fraction |
| A | GCC | 0.40 | P | CCC | 0.33 | V | GTG | 0.46 | R | CGG | 0.21 |
| A | GCT | 0.26 | P | CCT | 0.28 | V | GTC | 0.24 | R | AGA | 0.20 |
| A | GCA | 0.23 | P | CCA | 0.27 | V | GTT | 0.18 | R | AGG | 0.20 |
| A | GCG | 0.11 | P | CCG | 0.11 | V | GTA | 0.12 | R | CGC | 0.19 |
| | | | | | | | | | R | CGA | 0.11 |
| G | GGC | 0.34 | T | ACC | 0.36 | L | CTG | 0.40 | R | CGT | 0.08 |
| G | GGA | 0.25 | T | ACA | 0.28 | L | CTC | 0.20 | | | |
| G | GGG | 0.25 | T | ACT | 0.24 | L | CTT | 0.13 | S | AGC | 0.24 |
| G | GGT | 0.16 | T | ACG | 0.11 | L | TTG | 0.13 | S | TCC | 0.22 |
| | | | | | | L | CTA | 0.08 | S | TCT | 0.18 |
| | | | | | | L | TTA | 0.07 | S | AGT | 0.15 |
| | | | | | | | | | S | TCA | 0.15 |
| | | | | | | | | | S | TCG | 0.06 |
| | | | | | | | | | | | |

Anhand der Spezies-spezifischen codon usage table (CUT) werden nun successive alle ICCs von 5' nach 3' verändert und bit für bit die Zusatzinformation eingebracht. Dabei wird festgelegt:
Binäre 1 = erst- oder drittbestes Kodon
Binäre 0 = zweit- oder viertbestes Kodon

Die Gewichtung "erst-" .. "viertbestes" Kodon reflektiert dabei die Häufigkeit des jeweiligen Kodons, mit der es im Zielorganismus für die Kodierung seiner Aminosäure verwendet wird. Eine Datenbank hierüber ist zu finden unter: http://www.kazusa.or.jp/codon/.

Die Alternative von je zwei möglichen Kodons pro bit erlaubt in wohl allen Fällen eine Vermeidung von unerwünschten Sequenzmotiven bei der Optimierung. Weiterhin können natürlich auch ICC-benachbarte nicht-ICC Kodons verändert werden, um bestimmte Motive auszuschließen.

Für eine eindeutige Ver- und Entschlüsselung ist eine definierte CUT notwendig. Vor allem für wenig untersuchte Organismen werden sich jedoch CUTs zukünftig noch verändern. Daher ist in manchen Fällen eine Hinterlegung einer datierten CUT notwendig. Allerdings ist nur die Reihenfolge der ICC Codons relevant, nicht die tatsächlichen Zahlen deren Häufigkeit.

Diese Hinterlegung der Reihenfolge kann auf Papier oder notariell erfolgen. Möglich ist natürlich auch die Unterbringung dieser Daten in der DNA selber, beispielsweise der 3' UTR (unmittelbar hinter dem Gen). Für die Hinterlegung der ICC CUT werden 22 nt benötigt (siehe Beispiel 2).

Für die häufigsten Zielorganismen (Säugetiere, Kulturpflanzen, E. coli, Bäckerhefe etc.) sind die Kodon-Tabellen jedoch so vollständig, dass sie sich nicht mehr verändern werden.

Wenn zwei oder mehr Kodons die gleiche Häufigkeit in der CUT aufweisen, werden die betreffenden Kodons alphabetisch sortiert: A>C>G>T.

Das Ende einer Nachricht kann mit einem vereinbarten Stopp-Zeichen markiert werden, z.B. "11 1111", entsprechend dem underscore Zeichen.

Die Strategie, das erst- oder drittbestes Kodon als binäre 1, das zweit- oder viertbestes Kodon als binäre 0 zu definieren, also überhaupt mit einer codon usage table zu arbeiten, führt zu einem Gen, das erstens weitgehend optimiert ist, also im Zielorganismus gut funktioniert und zweitens ein Wasserzeichen zulässt.

Prinzipiell ist es alternativ dazu auch möglich, alle Aminosäuren, für die es zwei oder mehr Kodons gibt, als ICC zu definieren und als Kodierungsprinzip für die steganographische Dateneinbettung zu vereinbaren:
Binäre 1 = G oder C an Codonposition 3
Binäre 0 = A oder T an Codonposition 3

Möglich ist dies für die 18 Aminosäuren GEDAVRSKNTIQHPLCYF. (Im obigen auf Qualitätsranking basierenden Verfahren gibt es nur 8 ICCs.) So lässt sich mehr als doppelt soviel Information in einem Gen unterbringen und es muss in keinem Fall eine eindeutige CUT hinterlegt werden. Der Nachteil dieser Methode ist allerdings, dass das resultierende Gen nicht oder kaum optimiert sein wird.

Im vorliegenden Beispiel wurde die Nachricht "GENE" im N-Terminus der Telomerase von M. musculus verschlüsselt. Diese Nachricht enthält 4x6=24bit.

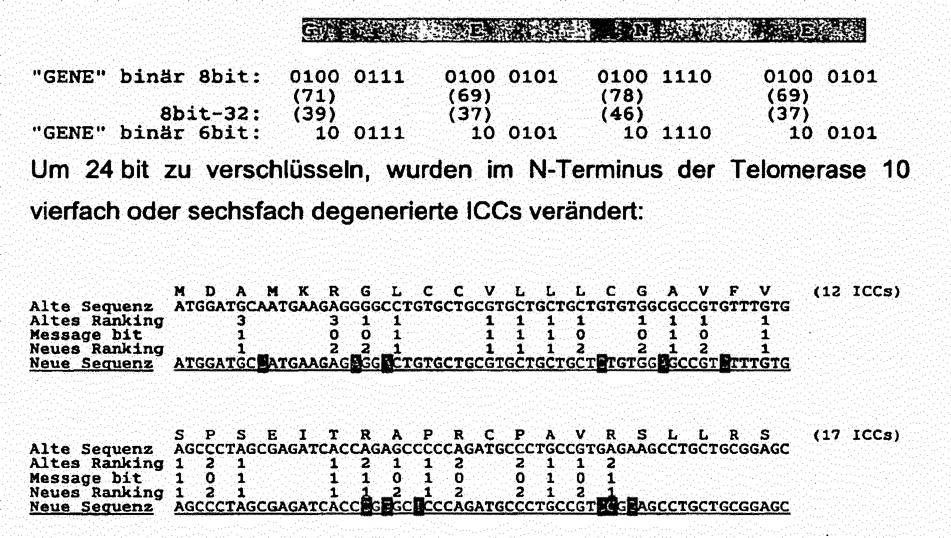

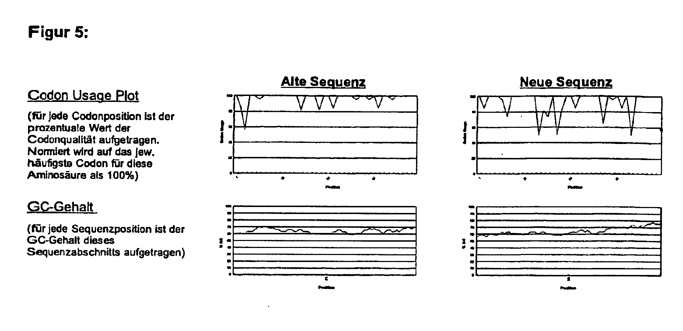

Während der Kodierung entstanden keine unerwünschten Motive oder zu hoher GC-Gehalt. Daher war es nicht notwendig, auf die dritt- und viertbesten Kodons zurückzugreifen. Ein Vergleich der Analyse der Ausgangssequenz und der modifizierten Sequenz ist in Figur 5 gezeigt.

### Beispiel 2: Verschlüsselung der Kodon Usage Tabelle für Escherichia coll und Hinterlegung als Nukleinsäuresequenz

Wesentlich für die Entschlüsselung der in die Gene eingebetteten Information ist die Kenntnis der verwendeten Kodierung. Sie ist der Schlüssel für die Dekodierung und kann bevorzugt aus der vom Organismus vorgegebenen Kodon Usage Tabelle bestehen. Prinzipiell kann der verwendete Schlüssel jedoch beliebig aus ca. 5.48 x 10¹⁹ möglichen Kombinationen gewählt werden.

Es ist möglich, diesen Schlüssel ebenfalls in Form einer spezifischen Nukleotidsequenz zu kodieren und so z.B. innerhalb des Genoms zu hinterlegen.

Zunächst wird die Kodon Usage Tabelle alphabetisch nach Aminosäuren, dann die Kodons einer Aminosäure alphabetisch nach Kodons sortiert:

| **Aminosäure** | **Kodon** | **Häufigkeit** | **Rang** |
|---|---|---|---|
| A | GCA | 0,22 | 3 |
| A | GCC | 0,27 | 2 |
| A | GCG | 0,35 | 1 |
| A | GCT | 0,16 | 4 |
| C | TGC | 0,55 | 1 |
| C | TGT | 0,45 | 2 |
| D | GAC | 0,37 | 2 |
| D | GAT | 0,63 | 1 |
| E | GAA | 0,68 | 1 |
| E | GAG | 0,32 | 2 |
| F | TTC | 0,42 | 2 |
| F | TTT | 0,58 | 1 |
| G | GGA | 0,12 | 4 |
| G | GGC | 0,38 | 1 |
| G | GGG | 0,16 | 3 |
| G | GGT | 0,33 | 2 |
| H | CAC | 0,42 | 2 |
| H | CAT | 0,58 | 1 |
| I | ATA | 0,09 | 3 |
| I | ATC | 0,40 | 2 |
| **I** | ATT | 0, 50 | 1 |
| K | AAA | 0,76 | 1 |
| K | AAG | 0,24 | 2 |
| L | CTA | 0,04 | 6 |
| L | CTC | 0,10 | 5 |
| L | CTG | 0,49 | 1 |
| L | CTT | 0,11 | 4 |
| L | TTA | 0,13 | 2 |
| L | TTG | 0,13 | 3 |
| M | ATG | 1,00 | 1 |
| N | AAC | 0,53 | 1 |
| N | AAT | 0,47 | 2 |
| P | CCA | 0,19 | 2 |
| P | CCC | 0,13 | 4 |
| P | CCG | 0, 51 | 1 |
| P | CCT | 0,17 | 3 |
| Q | CAA | 0, 33 | 2 |
| Q | CAG | 0,67 | 1 |
| R | AGA | 0,05 | 5 |
| R | AGG | 0,03 | 6 |
| R | CGA | 0,07 | 4 |
| R | CGC | 0,37 | 1 |
| R | CGG | 0,11 | 3 |
| R | CGT | 0,36 | 2 |
| S | AGC | 0,27 | 1 |
| S | AGT | 0,16 | 2 |
| S | TCA | 0,14 | 6 |
| S | TCC | 0,15 | 3 |
| S | TCG | 0,15 | 4 |
| S | TCT | 0,15 | 5 |
| T | ACA | 0,15 | 4 |
| T | ACC | 0,41 | 1 |
| T | ACG | 0,27 | 2 |
| T | ACT | 0,17 | 3 |
| V | GTA | 0,16 | 4 |
| V | GTC | 0,21 | 3 |
| V | GTG | 0,37 | 1 |
| V | GTT | 0,26 | 2 |
| W | TGG | 1,00 | 1 |
| Y | TAC | 0,43 | 2 |
| Y | TAT | 0,57 | 1 |
| Stopp | TAA | 0,59 | 1 |
| Stopp | TAG | 0,09 | 3 |
| Stopp | TGA | 0,32 | 2 |

Die Spalte "Häufigkeit" enthält den prozentualen Anteil des jeweiligen Kodons bezogen auf die jeweilige Aminosäure, die Spalte "Rang" enthält die Rangstufe des jeweiligen Kodons. Der Wert "Rang" definiert die Häufigkeit des jeweiligen Kodons innerhalb einer Aminosäure. Bei zwei oder mehr gleichen Häufigkeitswerten innerhalb einer Aminosäure werden die Rangstufen der gleichhäufigen Kodons zusätzlich alphabetisch vergeben. Die Spalte "Rang" enthält also den Schlüssel.

Im Beispiel haben die alphabetisch sortierten Kodons für Alanin (GCA, GCC, GCG, GCT) die Rangfolge 3,2,1,4 bzw. 3214.

Für Aminosäuren mit einem Kodon (M,W) gibt es nur eine Möglichkeit der Rangfolge (1).

Für Aminosäuren mit zwei Kodons (C,D,E,F,H,K,N,Q,Y) gibt es zwei Möglichkeiten der Rangfolge (12, 21).

Für Aminosäuren mit drei Kodons (I, Stopp) gibt es sechs Möglichkeiten der Rangfolge (123, 132, 213, 231, 312, 321).

Für Aminosäuren mit vier Kodons (A,G,P,T,V) gibt es 24 Möglichkeiten der Rangfolge (1234, 1243, 1324, ..., 4231, 4312, 4321).

Für Aminosäuren mit sechs Kodons (L,R,S) gibt es 720 Möglichkeiten der Rangfolge (123456, 123465, 123546, ..., 654231, 654312, 654321).

Aus diesen Zahlen geht hervor, dass 1² x 2⁹ x 6² x 24⁵ x 720³ = 5.48 x 10¹⁹ unterschiedliche Rangfolgenkombinationen existieren. Dies ist somit die Anzahl der möglichen Schlüssel.

Für jede Aminosäuregruppe (ein, zwei, drei, vier, sechs Kodons) wird eine aufsteigende Liste aller möglichen Rangfolgen angelegt und binär durchnummeriert. Dies ist für die 24 möglichen Rangfolgen der Aminosäuren mit vier Kodons (A,G,P,T,V) exemplarisch gezeigt:

| **Rangfolge** | **Dezimal** | **Binär** |
|---|---|---|
| 1234 | 00 | 00000 |
| 1243 | 01 | 00001 |
| 1324 | 02 | 00010 |
| 1342 | 03 | 00011 |
| 1423 | 04 | 00100 |
| 1432 | 05 | 00101 |
| 2134 | 06 | 00110 |
| 2143 | 07 | 00111 |
| 2314 | 08 | 01000 |
| 2341 | 09 | 01001 |
| 2413 | 10 | 01010 |
| 2431 | 11 | 01011 |
| 3124 | 12 | 01100 |
| 3142 | 13 | 01101 |
| 3214 | 14 | 01110 |
| 3241 | 15 | 01111 |
| 3412 | 16 | 10000 |
| 3421 | 17 | 10001 |
| 4123 | 18 | 10010 |
| 4132 | 19 | 10011 |
| 4213 | 20 | 10100 |
| 4231 | 21 | 10101 |
| 4312 | 22 | 10110 |
| 4321 | 23 | 10111 |

Für die binäre Kodierung der Rangreihenfolgen der Aminosäuren mit einem Kodon benötigt man 0 binäre Stellen.

Für die binäre Kodierung der Rangreihenfolgen der Aminosäuren mit zwei Kodons benötigt man 1 binäre Stelle (dezimal 0 = binär 0 & dezimal 1 = binär 1).

Für die binäre Kodierung der Rangreihenfolgen der Aminosäuren mit drei Kodons benötigt man 3 binäre Stellen (dezimal 0 = binär 000 & dezimal 5 = binär 101).

Für die binäre Kodierung der Rangreihenfolgen der Aminosäuren mit vier Kodons benötigt man 5 binäre Stellen (dezimal 0 = binär 00000 & dezimal 23 = binär 10111).

Für die binäre Kodierung der Rangreihenfolgen der Aminosäuren mit sechs Kodons benötigt man 10 binäre Stellen (dezimal 0 = binär 0000000000 & dezimal 719 = binär 1011001111).

Jeder Rangfolge der alphabetisch sortierten Aminosäuren lässt sich also eine spezifische binäre Zahl zuordnen. Die Gesamtheit der binären Zahlen repräsentiert die spezifische Kodon Usage Tabelle, die für das steganografische Verfahren verwendet wird.

| **Aminosäure** | **Rangfolge** | **Binär** | **nur 4er & 6er** |
|---|---|---|---|
| A | 3214 | 01110 | 01110 |
| C | 12 | 0 | |
| D | 21 | 1 | |
| E | 12 | 0 | |
| F | 21 | 1 | |
| G | 4132 | 10011 | 10011 |
| H | 21 | 1 | |
| **I** | 321 | 101 | |
| K | 12 | 0 | |
| L | 651423 | 1010111100 | 1010111100 |
| M | 1 | | |
| N | 12 | 0 | |
| P | 2413 | 01010 | 01010 |
| Q | 21 | 1 | |
| R | 564132 | 1001010011 | 1001010011 |
| S | 126345 | 0000010010 | 0000010010 |
| T | 4123 | 10010 | 10010 |
| V | 4312 | 10110 | 10110 |
| W | 1 | | |
| Y | 21 | 1 | |
| Stopp | 132 | 001 | |

Die gesamte 70-stellige binäre Folge der Kodon Usage Tabelle dieses Beispiels lautet demnach:
0111001011001111010101011110000101011001010011000001001010010 101101001

Um diese binäre Folge in eine Nukleotidsequenz zu übersetzen wird jeder Nukleobase ein fester zweistelliger Binärwert zugeordnet: A = 00, C = 01, G = 10, T = 11

Anhand dieses Schlüssels lässt sich die binäre Folge in eine 35-stellige Nukleotidsequenz übersetzen:
CTAGTATTCCCCTGACCCGCCATAACAGGCCCGGC

Werden bei der steganografischen Einbettung von Informationen in die kodierende Sequenz nur Aminosäuren mit vier oder sechs Kodons verwendet werden, ist es ausreichend, sich bei der Hinterlegung der Kodon Usage Tabelle auf diese Aminosäuren zu beschränken. Die relevanten Binärzahlen sind in obiger Tabelle in der Spalte "nur 4er & 6er" angegeben und ergeben zusammen die 56-stellige binäre Folge:
01110100111010111100010101001010011000001001010010101100

Anhand des oben erwähnten Schlüssels lässt sie sich in folgende 28-stellige Nukleotidsequenz übersetzen:
CTCATGGTTACCCAGGCGAAGCCAGGTA

Wie bereits erwähnt, lässt sich darüber hinaus die binäre Folge vor der Übersetzung in eine Nukleotidsequenz über konventionelle Verschlüsselungsalgorithmen mit einem Passwort verschlüsseln.

Die Rückübersetzung der Nukleotidsequenz in eine binäre Folge und eine Rangreihenfolge (Schlüssel) erfolgt in umgekehrter Reihenfolge analog zum beschriebenen Verfahren.

### Beispiel 3: Studie zur Expression von E.Coli

### Konstrukt eGFP(opt):

Der offene Leserahmen für *enhanced green fluorescent protein* (eGFP) wurde optimiert für die Expression in *E. coli.* Dabei wurde ein codon adaptation index (CAI) von 0,93 und ein GC-Gehalt von 53% erzielt.

### Konstrukt eGFP(msg):

Gemäß der Erfindung wurde in die optimierte DNA-Sequenz die Nachricht "AEQUOREA VICTORIA." eingebettet, wobei als Schlüssel die *codon usage table* (CUT) von *E. coli* zur Anwendung kam und nur solche Codons für die Unterbringung der Bits verwendet wurden, die einen Degenerationsgrad von 4 bzw. 6 haben, also für die Aminosäuren A,G,P,T,V,L,R,S codieren. Die Einbettung der 18 x 6 = 108 Bit langen Nachricht hat 71 Nukleotidaustausche zur Folge, ändert also die Sequenz zu 10%. Der CAI ändert sich auf 0,84, der GC-Gehalt auf 47%.

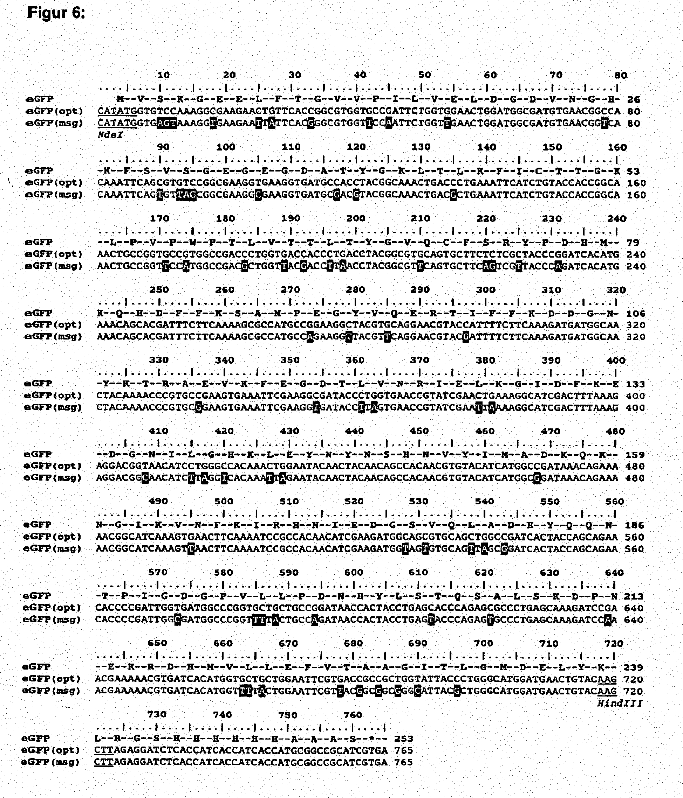

Ein Alignment der beiden Sequenzen eGFP(opt) und eGFP(msg) ist in Figur 6 gezeigt.

Beide Gene wurden synthetisch hergestellt und über NdeI/HindIII in den Expressionsvektor pEG-His ligiert. C-terminal erhalten die Proteine dadurch ein 6xHis-tag.

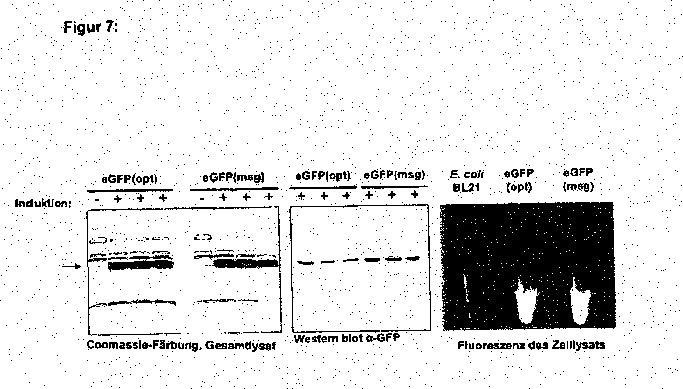

Beide Gene, eGFP(opt) und eGFP(msg) wurden in *E. coli* exprimiert und über Coomassie-Gel, Western Blot (mit einem GFP-spezifischen Antikörper) und Fluoreszenz analysiert. Die Ergebnisse sind in Figur 7 gezeigt. Es zeigte sich, dass eGFP(msg) etwa um den Faktor 2 besser exprimiert als eGFP(opt). Diese Expressionssteigerung ist ein zufälliger Effekt und nicht die Regel (gemäß Studien mit anderen Genen). Wichtig ist festzuhalten, dass die Expression unter der Einbettung der Nachricht nicht leidet.

### Beispiel 4: Studie zur Expression in humanen Zellen

### Konstrukt EMG1(opt):

Der offene Leserahmen für das humane Gen *EMG1 nucleolar protein homolog* wurde optimiert für die Expression in humanen Zellen. Dabei wurde ein codon adaptation index (CAI) von 0.97 und ein GC-Gehalt von 64% erzielt.

### Konstrukt EMG1(msg):

Gemäß der Erfindung wurde in die optimierte DNA-Sequenz die Nachricht *"GENEART AG PAT* US1234567*"* eingebettet, wobei als Schlüssel die *codon usage table* (CUT) von *H. sapiens* zur Anwendung kam und nur solche Codons für die Unterbringung der Bits verwendet wurden, die einen Degenerationsgrad von 4 bzw. 6 haben, also für die Aminosäuren A,G,P,T,V,L,R,S codieren. Die Einbettung der 24 x 6 = 144 Bit langen Nachricht hat 92 Nukleotidaustausche zur Folge, ändert also die Sequenz zu 12%. Der CAI ändert sich auf 0,87, der GC-Gehalt auf 59%.

### Konstrukt EMG1(enc):

Zunächst wurde die Nachricht "*GENEART AG PAT* US1234567" über das gängige polyalphabetische Vigenère-Verfahren (nach Blaise de Vigenère, 1586) mit dem Passwort "*Secret*" verschlüsselt, wodurch aus der Nachricht die Zeichenkette "*:JQWF&G%DY%$4Y#'XE%87G;K*" generiert wurde. Neben dem recht einfachen und unsicheren Vigenère-Verfahren, bei dem ein Klartextbuchstabe je nach seiner Position im Text durch verschiedene Geheimtextbuchstaben ersetzt wird, ist hier prinzipiell jedes andere Verschlüsselungsverfahren einsetzbar. Gemäß der Erfindung wurde in die optimierte DNA-Sequenz die verschlüsselte Zeichenkette "*:JQWF&G%DY%* $*4Y*#'*XE*%*87G;K*" eingebettet, wobei als Schlüssel die *codon usage table* (CUT) von *H. sapiens* zur Anwendung kam und nur solche Codons für die Unterbringung der Bits verwendet wurden, die einen Degenerationsgrad von 4 bzw. 6 haben, also für die Aminosäuren A,G,P,T,V,L,R,S codieren. Die Einbettung der 24 x 6 = 144 Bit langen Nachricht hat 93 Nukleotidaustausche zur Folge, ändert also die Sequenz zu 12%. Der CAI ändert sich auch hier auf 0.87, der GC-Gehalt auf 59%.

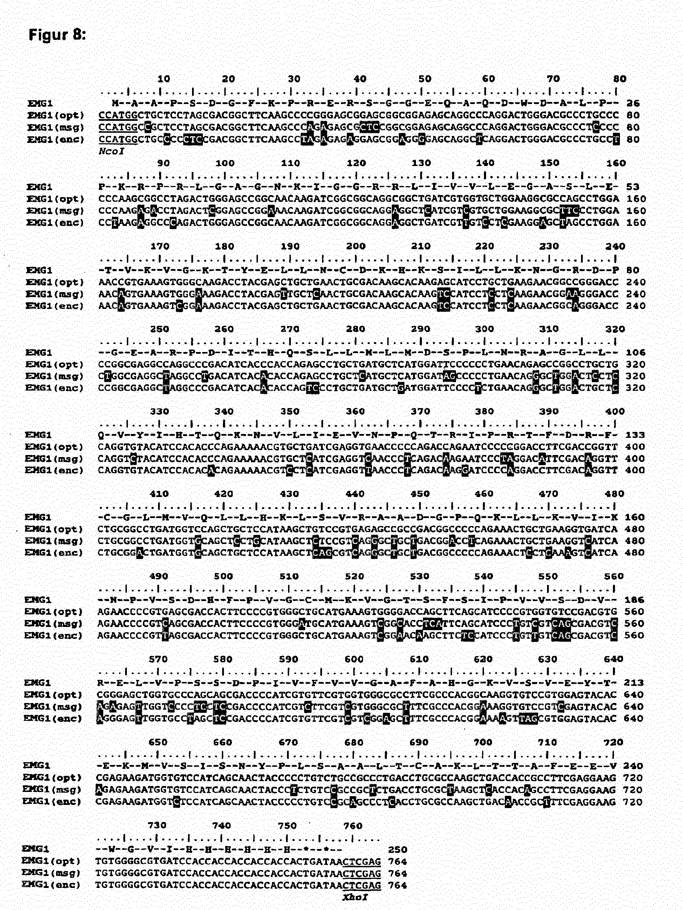

Ein Alignment der Sequenzen von EMG1 (opt), EMG1 (msg) und EMG1 (enc) ist in Figur 8 gezeigt.

Alle drei Gene wurden synthetisch hergestellt und über Ncol/Xhol in den Vektor pTriEx1.1 ligiert, der eine Expression in Säugerzellen ermöglicht.

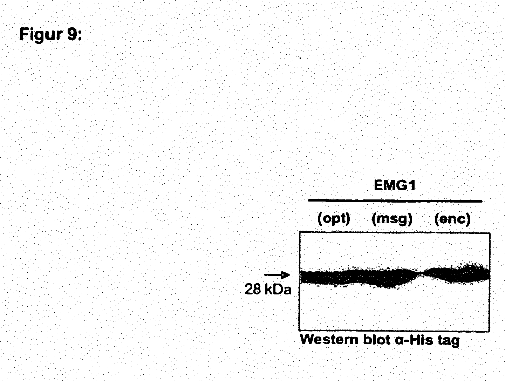

Humane HEK-293T Zellen wurden mit den drei Konstrukten EMG1(opt), EMG1(msg) und EMG1(enc) transfiziert und nach 36 h geerntet. Die Expression von EMG1 wurde über Western Blot Analyse (mit einem Hisspezifischen Antikörper) nachgewiesen. Alle drei Konstrukte zeigen eine Vergleichbare Expressionsstärke. Die Ergebnisse sind in Figur 9 gezeigt.

## Patentansprüche

1. Verfahren zum Entwerfen von Nukleinsäuresequenzen, in denen Informationen enthalten sind, welches die Schritte umfasst:
(a) Zuordnen eines ersten bestimmten Werts für mindestens ein erstes Nukleinsäure-Kodon aus einer Gruppe von degenerierten Nukleinsäure-Kodons, welche dieselbe Aminosäure kodieren,
Zuordnen eines zweiten bestimmten Werts für mindestens ein zweites Nukleinsäure-Kodon aus der Gruppe,
ggf. Zuordnen eines oder mehrerer weiterer Werte für jeweils mindestens ein weiteres Nukleinsäure-Kodon aus der Gruppe,
worin die ersten und zweiten und ggf. weitere Werte innerhalb der Gruppe von Kodons welche dieselbe Aminosäure kodieren mindestens jeweils einmal vergeben werden;
(b) Bereitstellen einer zu speichernden Information als Abfolge von n Werten, die jeweils ausgewählt sind aus ersten und zweiten und ggf. weiteren Werten;
(c) Bereitstellen einer Ausgangs-Nukleinsäuresequenz, wobei die Ausgangs-Nukleinsäuresequenz ein kodierender DNA-Strang ist, und wobei die Sequenz n degenerierte Kodons umfasst, denen gemäß (a) erste und zweite und ggf. weitere Werte zugeordnet sind, worin n eine ganze Zahl ≥ 1 ist; und
(d) Entwerfen einer modifizierten Sequenz der Nukleinsäuresequenz aus (c), worin an den Positionen der n degenerierten Kodons der Ausgangs-Nukleinsäuresequenz jeweils ein Nukleinsäure-Kodon aus der Gruppe degenerierter Kodons, die für dieselbe Aminosäure kodieren, ausgewählt wird, dem durch die Zuordnung aus (a) ein Wert entspricht, so dass die Abfolge der den n Kodons zugeordneten Werten die zu speichernde Information ergibt.

2. Verfahren nach Anspruch 1 , worin die Aminosäuren in Schritt (a) ausgewählt sind aus sechsfach kodierten Aminosäuren, wie Leucin, Serin, Arginin und/oder vierfach kodierten Aminosäuren, wie Alanin, Glycin, Valin, Prolin.

3. Verfahren nach Anspruch 1 oder 2, worin in Schritt (a) allen Kodons, welche für dieselbe Aminosäure bzw. Stopp kodieren, erste, zweite oder ggf. weitere Werte zugeordnet werden.

4. Verfahren nach einem der vorhergehenden Ansprüche, worin in Schritt (a) erste und zweite Werte aber keine weiteren Werte zugeordnet werden, und die Information in Schritt (b) in binärer Form bereitgestellt wird, und worin die ersten und zweiten Werte innerhalb der Gruppe von degenerierten Nukleinsäure-Kodons, welche dieselbe Aminosäure bzw. Stopp kodieren, jeweils mehrfach vergeben werden.

5. Verfahren nach einem der vorhergehenden Ansprüche, worin die Zuordnung eines ersten oder zweiten oder ggf. weiteren Werts zu einem Nukleinsäure-Kodon innerhalb der Gruppe von degenerierten Kodons, welche dieselbe Aminosäure bzw. Stopp kodieren, in Schritt (a) abhängig von der Häufigkeit des Gebrauchs des Kodons in einem bestimmten Organismus erfolgt.

6. Verfahren nach einem der vorhergehenden Ansprüche, worin die Ausgangs-Nukleinsäure für ein Polypeptid kodiert und die in Schritt (d) entworfene modifizierte Sequenz für dasselbe Polypeptid kodiert.

7. Verfahren nach einem der vorhergehenden Ansprüche, worin Anfang und/oder Ende der zu speichernden Information in dem Polynukleotidderivat markiert sind.

8. Verfahren nach einem der vorhergehenden Ansprüche, weiterhin umfassend den Schritt
(e) Herstellen der in Schritt (d) entworfenen modifizierten Sequenz, worin in die modifizierte Sequenz in Schritt (e) durch Mutation aus der Ausgangssequenz hergestellt wird, oder worin die modifizierte Sequenz in Schritt (e) synthetisch hergestellt wird.

9. Verfahren nach einem der vorhergehenden Ansprüche, worin die zu speichernde Information verschlüsselt wird, bevor sie in eine Abfolge von n Werten umgewandelt wird.

10. Verfahren nach einem der vorhergehenden Ansprüche, worin ein Schlüssel für
die Zuordnung gemäß Schritt (a) selbst verschlüsselt und in einer Nukleinsäure gespeichert wird, worin der Schlüssel in dem Nukleinsäure-Derivat aus Schritt (d) oder in einer anderen Nukleinsäure gespeichert wird.

11. Verfahren nach Anspruch 8 , weiterhin umfassend den Schritt:
(f) Einbringen der modifizierten Nukleinsäure in einen Vektor.

12. Verfahren nach einem der Ansprüche 8 oder 11, weiterhin umfassend den Schritt:
(g) Einbringen der modifizierten Nukleinsäure oder des Vektors in eine Zelle.

13. Verfahren nach einem der Ansprüche 8, 11 oder 12 weiterhin umfassend den Schritt:
(h) Übermittlung einer gewünschten Information durch Übermitteln der Nukleinsäuresequenz oder der Nukleinsäure, des Vektors und/oder der Zelle an einen gewünschten Empfänger, und worin die Nukleinsäure, der Vektor, und/oder die Zelle ggf. vor der Übermittlung an den Empfänger mit anderen Nukleinsäuren, Vektoren bzw. Zellen vermischt wird, welche die gewünschte Information nicht enthalten.

14. Verfahren nach einem der Ansprüche 8 oder 11 bis 13, wobei die in Schritt (e) hergestellte, modifizierte Nukleinsäuresequenz zur Kennzeichnung von Genen, und/oder Zellen verwendet wird.

## Claims

1. A method for creating nucleic acid sequences containing information, which comprises the steps:
(a) Assigning a first defined value for at least one first nucleic acid codon from a group of degenerate nucleic acid codons which encode the same amino acids,
Assigning a second defined value for at least one second nucleic acid codon from the group,
Possibly assigning one or more additional values, each for at least one additional nucleic acid codon from the group,
wherein the first and second and possibly additional values within the group of codons which code for the same amino acid are each assigned at least once;
(b) providing information to be stored as a series of n values, each selected from among the first and second and possibly additional values;
(c) providing a starting nucleic acid sequence, the starting nucleic acid sequence being a coding DNA strand, the sequence including n degenerate codons to which first and second and possibly additional values are assigned according to (a), where n is an integer ≥ 1; and
(d) Creating a modified sequence of the nucleic acid sequence from (c) wherein, at the positions of the n degenerate codons of the starting nucleic acid sequence, one nucleic acid codon each from the group of degenerate codons encoding the same amino acid is selected, corresponding to a value due to the assignment in (a), so that the series of values assigned to the n codons yields the information to be stored.

2. The method according to Claim 1 wherein the amino acids in step (a) are selected from six fold encoded amino acids such as leucine, serine, arginine, and/or four fold encoded amino acids such as alanine, glycine, valine, proline.

3. The method according to Claim 1 or 2, wherein in step (a) first, second or possibly further values are assigned to all codons [handwritten] which encode the same amino acid or stop.

4. The method according to one of the foregoing claims, wherein in step (a) first and second values, but no additional values are assigned, and the information in step (b) is provided in binary form, and wherein the first and second values within the group of degenerate nucleic acid codons, which encode the same amino acid or stop, are assigned repeatedly.

5. The method according to one of the foregoing claims, wherein the assignment of a first or second or possible additional value to a nucleic acid codon within the group of degenerate codons, which encode the same amino acid or stop, occurs in step (a) depending on the frequency of use of the codon in a particular organism.

6. The method according to one of the foregoing claims, wherein the starting nucleic acid encodes a polypeptide and the modified sequence created in step (d) encodes the same polypeptide.

7. The method according to one of the foregoing claims, wherein the beginning and/or the end of the information to be stored is marked in the polynucleotide derivative.

8. The method according to one of the foregoing claims, further comprising the step:
(e) producing the modified sequence created in step (d), wherein the modified sequence in step (e) is produced by mutation from the starting sequence, or wherein the modified sequence in step (e) is made synthetically.

9. The method according to one of the foregoing claims, wherein the information to be stored is encrypted before it is converted into a series of n values.

10. The method according to one of the foregoing claims, wherein a key for the assignment according to step (a) is itself encrypted and stored in a nucleic acid, wherein the key is stored in the nucleic acid derivative from step (d) or in another nucleic acid.

11. The method according to Claim 8, further comprising the step:
(f) Inserting the modified nucleic acid into a vector.

12. The method according to one of Claims 8 or 11, further comprising the step:
(g) Inserting the modified nucleic acid or the vector into a cell.

13. The method according to one of Claims 8, 11 or 12, further comprising the step:
(h) Transmitting desired information by transmitting the nucleic acid sequence or the nucleic acid, the vector and/or the cell to an intended recipient, and wherein the nucleic acid, the vector and or the cell is mixed if necessary with other nucleic acids, vectors or cells which do not contain the desired information.

14. The method according to one of Claims 8 or 11 through 13, wherein the modified nucleic acid sequence produced in step (e) is employed for labeling genes and/or cells.

## Revendications

1. Procédé de conception de séquences d'acides nucléiques contenant des informations, comprenant les étapes consistant à :
(a) Attribuer une première valeur déterminée à au moins un premier codon d'acide nucléique choisi dans un groupe de codons d'acides nucléiques dégénérés qui codent le même acide aminé,
attribuer une deuxième valeur déterminée à au moins un deuxième codon d'acide nucléique du groupe,
le cas échéant attribuer une ou plusieurs valeurs supplémentaires à chacun des au moins un codon d'acide nucléique supplémentaires du groupe,
dans lequel les première et deuxième, et le cas échéant les valeurs supplémentaires à l'intérieur du groupe de codons qui codent le même acide aminé sont attribuées chacune au moins une fois ;
(b) Préparer une information à enregistrer sous la forme d'une succession de valeurs n, qui sont choisies chacune parmi la première et la deuxième valeur et le cas échéant les valeurs supplémentaires ;
(c) Préparer une séquence d'acide nucléique initiale, la séquence d'acide nucléique de départ étant un brin d'ADN codant, et dans laquelle la séquence contient n codons dégénérés, auxquels sont attribuées une première et une deuxième valeur en conformité avec (a), et le cas échéant des valeurs supplémentaires, avec n étant un nombre entier ≥ 1 ; et
(d) Concevoir une séquence modifiée de la séquence d'acide nucléique de (c) dans laquelle est choisi chaque fois aux positions des n codons dégénérés de la séquence d'acide nucléique initiale, un codon d'acide nucléique du groupe de codons dégénérés qui codent le même acide aminé, auquel correspond par l'attribution, en conformité avec (a), une valeur, de manière à ce que la succession des valeurs attribuées aux n codons fournisse l'information à enregistrer.

2. Procédé selon la revendication 1, dans lequel les acides aminés de l'étape (a) sont choisis parmi des acides aminés à six possibilités de codage comme la leucine, la sérine, l'arginine et/ou des acides aminés à quatre possibilités de codage comme l'alanine, la glycine, la valine, la proline.

3. Procédé selon la revendication 1 ou 2, dans lequel à l'étape (a) tous les codons [rayé :]5 qui codent le même acide aminé ou l'arrêt, sont affectés d'une première, d'une deuxième valeur ou le cas échéant de valeurs supplémentaires.

4. Procédé selon l'une des revendications précédentes, dans lequel une première et une deuxième valeur, mais pas de valeur supplémentaire, sont affectées à l'étape (a) et l'information de l'étape (b) est préparée sous forme binaire, et dans lequel la première et la deuxième valeur à l'intérieur du groupe des codons dégénérés qui codent le même acide aminé ou l'arrêt, sont attribuées chacune plusieurs fois.

5. Procédé selon l'une des revendications précédentes, dans lequel l'attribution d'une première ou d'une deuxième valeur ou le cas échéant de valeurs supplémentaires à un codon d'acide nucléique à l'intérieur du groupe des codons dégénérés qui codent le même acide aminé ou l'arrêt, s'effectue à l'étape (a) en fonction de la fréquence d'utilisation du codon dans un organisme donné.

6. Procédé selon l'une des revendications précédentes, dans lequel l'acide nucléique initial code un polypeptide et la séquence modifiée conçue à l'étape (d) code le même polypeptide.

7. Procédé selon l'une des revendications précédentes, dans lequel le début et/ou la fin de l'information à enregistrer sont marqués dans le dérivé de polynyucléotide.

8. Procédé selon l'une des revendications précédentes comportant en outre l'étape consistant à
(e) Produire la séquence modifiée conçue à l'étape (d), la séquence modifiée dans l'étape (e) étant produite par mutation de la séquence initiale, ou bien la séquence modifiée dans l'étape (e) étant produite par synthèse.

9. Procédé selon l'une des revendications précédentes, dans lequel l'information à enregistrer est codée, avant d'être transformée en une succession de valeurs n.

10. Procédé selon l'une des revendications précédentes, dans lequel un code pour l'affectation conformément à l'étape (a) est automatiquement codé et stocké dans un acide nucléique, tandis que le code est stocké dans le dérivé d'acide nucléique de l'étape (d) ou dans un autre acide nucléique.

11. Procédé selon la revendication 8, comprenant en outre l'étape consistant à :
(f) Incorporer l'acide nucléique modifié dans un vecteur.

12. Procédé selon l'une des revendications 8 ou 11, comprenant en outre l'étape consistant à :
(g) Introduire l'acide nucléique modifié ou le vecteur dans une cellule.

13. Procédé selon l'une des revendications 8, 11 ou 12 comprenant en outre l'étape consistant à :
(h) Transmettre une information souhaitée en introduisant la séquence d'acide nucléique ou l'acide nucléique, le vecteur et/ou la cellule dans un receveur sélectionné, et dans lequel l'acide nucléique, le vecteur, et/ou la cellule sont le cas échéant mélangés avant l'introduction dans le receveur, avec d'autres acides nucléiques, vecteurs ou cellules qui ne contiennent pas l'information souhaitée.

14. Procédé selon l'une des revendications 8 ou 11 à 13, dans lequel la séquence d'acide nucléique modifiée produite à l'étape (e) est utilisée pour la caractérisation de gènes et/ou de cellules.
